Europäisches Patentamt

**European Patent Office**

**Office européen des brevets**

(11) Publication number: **0 235 805**
A2

# (12) EUROPEAN PATENT APPLICATION

(21) Application number: **87103041.7**

(22) Date of filing: **04.03.87**

(51) Int. Cl.⁴: **A 61 K 39/395, A 61 M 3/00**

(30) Priority: **04.03.86 GB 8605316**

(43) Date of publication of application: **09.09.87**
**Bulletin 87/37**

(84) Designated Contracting States: **AT BE CH DE ES FR GB GR IT LI LU NL SE**

(71) Applicant: **THE ROYAL FREE HOSPITAL SCHOOL OF MEDICINE, Rowland Hill Street, London NW3 2PF (GB)**

(72) Inventor: **Janossy, George, 23 Whitehall Road, Harrow Middlesex (GB)**

(74) Representative: **Grubb, Philip William, c/o B.A. Yorke & Co 98 The Centre, Feltham Middlesex TW13 4EP (GB)**

(54) Improvements relating to Immunosuppression.

㊼ A cocktail of anti T-cell monoclonal antibodies of different specificity, of which at least one is of the IgG class, and at least one is of the IgM class, is more effective in immunosuppression of organ transplant recipients than individual monoclonal antibodies.

EP 0 235 805 A2

IMPROVEMENTS RELATING TO IMMUNOSUPPRESSION

This invention relates to immunosuppression and more particularly, is concerned with the use of certain combinations of monoclonal antibodies in bringing about immunosuppression in a mammalian host.

In organ transplant surgery, particularly kidney, liver, heart and lung transplant surgery, it is necessary to suppress the immune system of the graft recipient to minimise the likelihood of graft rejection after surgery. Various immunosuppressive drugs have been proposed for this purpose but their use has to be carefully controlled since, in addition to undesirable side-effects arising from the use of certain immunosuppressive agents, there is also the difficulty that the immunosuppressive action makes the graft recipient particularly susceptible to infection by bacteria and viruses that would be controlled by a normal immune system. Immunosuppressive agents that have been used successfully in clinical practice include steroids, azathioprine and cyclosporin A. It is necessary in clinical practice to attempt to balance the degree of immunosuppression necessary to prevent or treat graft rejection episodes with the retention of a certain amount of the recipient's immune system to combat other infectious agents and, at the same time, to keep any possible undesirable side-effects under control.

In addition to the use of immunosuppressive drugs, attention has also focussed upon the use of certain monoclonal antibodies to suppress immune reactions, in particular, attention has been paid to monoclonal antibodies that recognise various surface antigens of T-cells. Here too, problems have been encountered in clinical practice, namely that single antibodies were not sufficiently effective, and/or caused severe side effects such as high fever.

It has now been found that beneficial results can be achieved by the use, in combination, of known monoclonal antibodies of different types.

Accordingly, the present invention provides a composition comprising a mixture of monoclonal antibodies to T-cell surface antigens, the mixture comprising at least one antibody of the IgG type and at least one antibody of the IgM type, said antibodies differing in specificity.

The invention further provides at least one monoclonal antibody against T-cell surface antigens of the IgG type and at least one monoclonal antibody against T-cell surface antigens of the IgM type, in association with one another for use in immunosuppression of the mammalian system, said antibodies differing in specificity.

It is believed that, in clinical practice, the monoclonal antibody of the IgG type will effectively reach the lymphoid and transplanted tissue, where it inactivates tissue-located T-cells while the antibody of the IgM type effectively inactivates the circulating T-cells in the mammalian host.

By "differing in specificity" is meant that although the antibodies of both types recognise T-cell surface antigens, they each recognise a different antigen or a different epitope of the same antigen. This means that the IgG and the IgM antibodies are not competing with each other for the same binding site on the T-cells.

The two types of antibody can be used in clinical practice in various ways. Preferably, the two types of antibody are mixed together and the physical mixture of antibodies of the two types, IgG and IgM, administered to the patient. An alternative procedure is the administration of the IgG antibody and the IgM antibody to the recipient from separate reservoirs but at the same time.

Although it is possible to produce the composition according to the invention as a dry lyophilised mixture of the components, a more preferred form of the composition according to the invention is an injectable solution containing both types of monoclonal antibody dissolved in a suitable aqueous carrier for example sterile water for injection or sterile buffered physiological saline. Preferably the solution is made up in vials each containing one unit dose of the composition, and these may be kept in frozen condition, for example by packing in dry ice or storage in a freezer.

It may be desirable to make up a solution of larger volume for administration by infusion rather as a bolus injection, and when this is done it is preferred to incorporate human serum albumin or the patient's own heparinised blood into the saline at the time of formulation. The presence of an excess of such physiologically inert protein prevents loss of monoclonal antibody by adsorption on to the walls of the container and tubing used with the infusion solution. If albumin is used, a suitable concentration is from 0.5 to 4.5 % by weight of the saline solution.

To aid in making up suitable compositions, the monoclonal antibodies may be packaged in separate vials within the same container, with instructions for mixing or concomitant administration. Alternatively, a twin-barrelled syringe may be provided, one barrel containing a solution of a monoclonal antibody of type IgG and the other containing a monoclonal antibody of type IgM.

The antibodies are administered parenterally, normally intravenously, for example into the antecubital or other peripheral vein.

In clinical practice, one normally aims to administer slighter larger amounts of IgM than IgG, e.g. in 2:1 weight ratio although the relative amounts can vary between 10:1 and 1:5, preferably between 5:1 and 1:1, on a weight basis.

Investigations so far indicate that the administration of these two types of antibody in combination is free from unacceptable side-effects at the dosage levels employed, and that there is no potentiation of the side-effects observed with the individual antibodies. The dosage levels are such that, when murine monoclonal antibodies are used, it is possible to detect immunoglobulin of murine origin in the patient's serum by conventional enzyme-linked immunosorbent assay (ELISA) or radioimmuno assay. This target dosage will normally call for the administration of the order of 0.1 − 0.2 milligram IgG antibody per kilogram body weight and 0.2 − 0.4 milligram IgM antibody per kilogram body weight of the patient. This, in turn, having regard to the volume of saline that it is desired to administer to the patient, will control the concentration of monoclonal antibody in the saline for administration. Practical requirements point to the formulation of saline containing about 2 − 10, preferably 2 − 4 mg/ml of the antibody of the IgG type and 4 − 20, preferably 4 − 8 mg/ml antibody of the IgM type when the antibodies are to be administered by bolus injection. When administration by infusion is indicated, the concentrations will be lower by a factor of from 20 to 200.

Murine monoclonal antibodies suitable for use in the present invention are known per se. Many monoclonal antibodies against T-cell surface antigens are available from Culture Collections in various countries of the world and specifically, the American Type Culture Collection of Rockville, Maryland, USA can provide suitable monoclonal antibodies or hybridomas secreting such antibodies. Hybridomas secreting monoclonal antibodies against T-cell surface antigens that can be used in the present invention and that are available from the ATCC include those available under the ATTC deposit numbers:

CRL 8000, CRL 8001, CRL 8002, CRL 8013, CRL 8016, CRL 8020, CRL 8014, CRL 8021, CRL 8022, CRL 8027, HB 2.

Other suitable monoclonal antibodies are available from the Collection of Antibodies that are circulated in connection with the International Workshops and Conferences on Human Leucocyte Differentiation Antigens. The second such Workshop and Conference was held in Boston, Massachusetts, USA in September 1984 at which a collection of monoclonal antibodies, submitted by each of the participating laboratories, was circulated to all participating laboratories for unrestricted use by themselves and their colleagues.

The Symposium Report of the Boston Workshop, subsequently published in "Leucocyte Typing II, Vol. I Human T Lymphocytes" by Reinherz, Haynes, Nadler and Bernstein, Springer Verlag, 1985, lists the various monoclonal antibodies discussed at that Conference which were circulated to and are available from any one of the participating laboratories around the world working in this area. As IgM antibody, use can be made for example of any one of the pan-T monoclonal antibodies available through the Boston Workshop Laboratories which are classified under their CD2, CD3 or CD6 cluster. The CD6 cluster includes 3 different monoclonal antibodies of the IgM type that recognise T-cell surface antigens having a molecular weight in the region of 120 kD, for example T12/3Pt 12B8 or RFT 12. The CD3 cluster, which is preferred, includes the antibody T10 B9, and the CD2 cluster includes the antibody 9-2.

Preferred monoclonal antibodies of the IgG type are those classified in the CD 7 or CD25 cluster of the Boston Workshop listing. The antibodies in the CD7 group react with a T-lineage associated antigen which is particularly strongly expressed on activated T-cells, having a molecular weight of about 40 kD, and show a specific affinity for activated T-cells. The antibodies in the CD25 group react with a T-cell activation antigen. The use of antibodies of this type has the result that some but not all of the tissue T-cells are inactivated, leaving a small residual seed of resting normal T-cells, which is beneficial from the clinical point of view.

Preferred IgG monoclonal antibodies of the CD7 or CD25 cluster are those of the $IgG_2$ subclass, particularly of the $IgG_{2a}$ subclass. A suitable antibody from the CD25 cluster is that designated Tac. From the CD7 cluster the preferred antibodies are designated RFT-2 and T-3Al and RFT-2 is the most preferred antibody of the IgG class for use in the present invention. A particularly preferred composition according to the invention comprises a mixture of the antibodies T10 B9 and RFT-2.

Although most of the available anti-T cell monoclonal antibodies are murine antibodies made by fusing mouse myeloma cells with spleen cells from a mouse immunised with human T cells, the invention is not limited to mouse monoclonals, and includes the use of IgG and IgM monoclonals which are derived from other animal species, or which are chimeric, e.g. part mouse and part human, or wholly human. Such antibodies may be produced by cell fusion techniques or directly by recombinant DNA technology. Preferred chimeric antibodies have the variable regions of the heavy and light chains derived from murine monoclonal antibodies of the CD clusters mentioned above, the rest of the molecule being human-derived.

As indicated above, it is preferred to administer a mixture of the IgG and IgM antibodies from a single saline solution or, alternatively, to inject a saline solution of IgG antibody and a saline solution of IgM antibody simultaneously or subsequently. In accordance with one specific protocol, 10 mg of RFT-2, an IgG monoclonal antibody, can be administered on the first day and 5 mg of RFT-2 can be administered daily for the next 6 - 10 days. At the same time as RFT-2 is administered, 20 mg of T10 B9, an IgM antibody, can be administered on the first day and 10 mg of T10 B9 can be given daily for the next 6 - 10 days.

It is possible to use the combination monoclonal antibody therapy of this invention as an isolated procedure, for example for patients who have been treated previously with other immunosuppressive agents but who are beginning to show signs of graft rejection. However, it is possible and usually desirable to use the monoclonal antibody combination of the present invention in association with other known immunosuppressive agents. For example, a typical regime may involve preparing a patient, prior to surgery, by treatment with an immunosuppressive drug which treatment is continued for several days or several weeks after the transplant surgery has taken place. In such cases, the combined therapy with the IgG and IgM monoclonal antibodies can take place at the same time as the treatment with the other immunosuppressive agent.

The combination therapy of the present invention can also be used with patients who are receiving their second, third or subsequent kidney graft when the antibody of IgG and IgM class can be given together from the day of transplant as a preventative treatment prior to the development of rejection episodes.

The combination therapy of the invention can also be used for patients receiving heart transplants and particularly those receiving a combined heart and lung transplant. In such cases, the antibodies can be administered either together or from separate injections. In this type of surgery, lymph nodes are usually transferred with the lung and the grafted lymphocytes cause severe side effects in the new host. These side effects can be inhibited by the use of the antibody therapy in accordance with the present invention as a preventative measure.

In addition to immunosuppression in conjunction with solid organ transplantation, the combination therapy of the invention may be used to treat patients receiving bone marrow transplants, either before or during the transplantation.

Other indications include the treatment of autoimmune diseases, for example rheumatoid arthritis (particularly juvenile-onset rheumatoid arthritis) or systemic lupus erythrematosis. In treatment of autoimmune diseases, the combination antibody therapy of the invention may be particularly useful in conjunction with administration of another immunosuppressant, for example cyclosporin A.

The following Examples illustrate the invention.

EXAMPLE 1:

The patients selected for therapy were those who had undergone kidney transplantation and developed rejection episodes in spite of ongoing immunosuppression by cyclosporin-A, steroid and azathioprine. These patients were treated with the mixture of monoclonal antibodies in accordance with this invention as a steroid sparing therapy.

The monoclonal antibodies used were RFT-2 which is an $IgG_2$ antibody from the CD7 cluster of the Boston Workshop and T10 B9 which is an IgM monoclonal antibody from the CD3 cluster of the Boston Workshop. The monoclonal antibodies were formulated in a saline solution at a concentration of 5 mg protein/ml. 1 ml of RFT-2 solution and 3 ml of T10 B9 solution were mixed together and this mixture then dispersed into 100 ml sterile buffered saline containing 4.5 % wt. of human albumin. This saline dispersion of RFT-2 and T10 B9 was infused intravenously into the patient over a 1 hour period. Infusion was continued at a rate such that the patient received a total of 10 mg of RFT-2 and 30 mg of T10 B9 on day one. Thereafter 5 mg of RFT-2 and 15 mg of T10 B9 was given daily for 7 - 9 days as a bolus injection. Therapy with the mixture of antibodies in accordance with this invention was found to inhibit rejection episodes.

EXAMPLE 2:

Example 1 was repeated using, in place of T10 B9, RFT-12, an IgM monoclonal antibody from the CD6 cluster of the Boston Workshop. The hybridoma producing RFT-12 was deposited at the European Collection of Animal Cell Cultures, Porton Down, on 2 March 1987 under the deposit number 87030201 . 10 mg of RFT-2 and 10 mg RFT-12 was given on day one, and 5 mg of RFT-2 and 10 mg of RFT-12 daily thereafter for 7-9 days.

Claims for Belgium, France, Germany, Italy, Luxemburg, Netherlands, Sweden, Switzerland, United Kingdom

1. A composition comprising a mixture of monoclonal antibodies to T-cell surface antigens, the mixture comprising at least one antibody of the IgG type and at least one antibody of the IgM type, said antibodies differing in specificity.

2. A composition according to Claim 1 in which at least one antibody is of the $IgG_2$ subclass.

3. A composition according to Claim 2 in which the IgG antibody is RFT-2.

4. A composition according to any one of Claims 1 – 3 in which the IgM antibody is T10 B9.

5. A composition according to any one of the previous claims in which the weight ratio of IgM to IgG antibodies is from 10:1 to 1:5.

6. A composition according to Claim 5 in the form of a solution in sterile saline for injection containing from 2–10 mg/ml IgG antibody and from 4–20 mg/ml IgM antibody.

7. A composition according to Claim 5 in the form of a solution in sterile saline for infusion containing 0.01 – 0.5 mg/ml of IgG antibody and from 0.02 – 1 mg/ml of IgM antibody, together with 0.5 – 4.5 % wt. human serum albumin or heparinized human blood.

8. At least one monoclonal antibody against T-cell surface antigens of the IgG type and at least one monoclonal antibody againstT-cell surface antigens of the IgM type in association with each other for use in immunosuppression of the mammalian system, said antibodies differing in specificity.

9.    A twin-pack containing separate unit dose forms of at least two monoclonal antibodies to T-cell surface antigen, at least one antibody being of the IgG type and at least one antibody of the IgM type said antibodies differing in specificity together with instructions for mixing or concomitant administration.

10.    A double-barrelled syringe containing, in one barrel, a solution of a monoclonal antibody of IgG type to a T-cell surface antigen and, in the other barrel, a solution of a monoclonal antibody of IgM type to a T-cell surface antigen, said antibodies differing in specificity.

Claims for Austria, Greece, Spain 1. A method for the production of a synergistic composition for immunosuppression comprising a mixture of monoclonal antibodies to T-cell surface antigens, comprising mixing together at least one antibody of the IgG type and at least one antibody of the IgM type, said antibodies differing in specificity.

2. A method according to Claim 1 in which at least one antibody is of the $IgG_2$ subclass.

3. A method according to Claim 2 in which the IgG antibody is RFT-2.

4. A method according to Claim 1 in which the IgM antibody is T10 B9.

5. A method according to Claim 1 in which the weight ratio of IgM to IgG antibodies is from 10:1 to 1:5.

6. A method according to Claim 5 in which the antibodies are dissolved in sterile saline to give a solution for injection containing from 2-10 mg/ml IgG antibody and from 4-20 mg/ml IgM antibody.

7. A method according to Claim 5 in which the antibodies are dissolved in sterile saline to give a solution for infusion containing 0.01 - 0.5 mg/ml of IgG antibody and from 0.02 - 1 mg/ml of IgM antibody, together with 0.5 - 4.5 % wt. human serum albumin or heparinized human blood.

3700/PG/FZ